# EUROPÄISCHE PATENTSCHRIFT

(11) **EP 0 418 797 B1**
(45) Veröffentlichungstag und Bekanntmachung des Hinweises auf die Patenterteilung: **24.08.1994**
(21) Anmeldenummer: 90117894.7
(22) Anmeldetag: 18.09.1990
(51) Int. Cl.: C07D 239/28, A61K 31/505

(54) **Pyrimidin-4,6-dicarbonsäurediamide, Verfahren zu deren Herstellung sowie Verwendung derselben sowie Arzneimittel auf Basis dieser Verbindungen**
Pyrimidin-4,6-dicarboxilic acid diamides, process for their preparation also medicine based on those compounds
Pyrimidines-4,6-dicarboxamide, procédé pour leur préparation et leur utilisation comme médicaments sur la base de ces composés

(30) Priorität: 21.09.1989 DE 3931432
(43) Veröffentlichungstag der Anmeldung: 27.03.1991
(73) Patentinhaber: HOECHST AKTIENGESELLSCHAFT, 65926 Frankfurt am Main (DE)
(72) Erfinder: Baader, Ekkehard, Dr., D-6240 Königstein/Taunus (DE); Bickel, Martin, Dr., D-6380 Bad Homburg (DE); Günzler-Pukall, Volkmar, Dr., D-3550 Marburg (DE); Henke, Stephan, Dr., D-6238 Hofheim am Taunus (DE)

(56) Entgegenhaltungen:
- EP-A- 0 112 617

## Beschreibung

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie α,α'-Dipyridyl zu einer Hemmung der C1_{q}-Biosynthese von Makrophagen führt (W. Müller et al., FEBS Lett. 90 (1978), 218; Immunbiology 155 (1978), 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß das Enzym Prolinhydroxylase durch Pyridin-2,4- oder -2,5-dicarbonsäure effektiv gehemmt wird (K. Majamaa et al., Eur. J. Biochem. 138 (1984), 239-245). Diese Verbindungen sind in der Zellkultur allerdings nur in sehr hoher Konzentrationen als Hemmstoffe wirksam (Tschank, G. et al., Biochem. J. 238 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und-2,5-dicarbonsäure, die die Kollagenbiosynthese im Tiermodell wirksam hemmen.

So wird in der DE-A 37 03 959 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In den deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamid vorgeschlagen. Die deutsche Patentanmeldung P 3924093.2 schlägt neue N,N'-Bis(alkoxyalkyl)-pyridin-2,4-dicarbonsäurediamide vor.

Überraschend wurde nun gefunden, daß Pyrimidin-4,6-dicarbonsäurediamide der Formel I 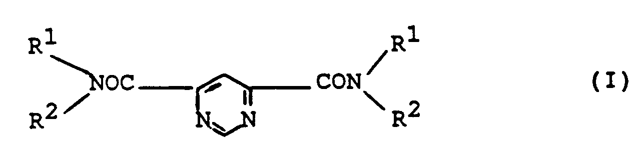
worin
- R¹: C₁ -C₁₂-Alkyl, C₂-C₁₂-Alkenyl oder C₂-C₁₂-Alkinyl bedeutet, welche unsubstituiert oder einfach oder im Falle der C₂-C₁₂ -Alkyle, C₂-C₁₂-Alkenyle und C₂-C₁₂-Alkinyle auch mehrfach substituiert sind mit
Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, oder mit
Indolyl oder Phenyl, welches unsubstituiert ist oder mit Halogen, Nitro, C₁-C₄-Alkyl oder C₁-C₄-Alkoxy 1-, 2-, oder 3-fach substituiert ist, wobei bei Mehrfachsubstitutionen die Substituenten auch voneinander unabhängig verschieden sein können
oder
- R¹: gesättigtes C₅-C₇-Cycloalkyl bedeutet, welches gegebenenfalls benzoanneliert ist,
oder
- R¹: Aryl oder Heteroaryl bedeutet, welches unsubstituiert oder seinerseits mit Halogen, Nitro, Cyano, C₁-C₄-Alkyl oder C₁-C₄-Alkoxy 1-, 2-, oder 3-fach substituiert ist, wobei bei Mehrfachsubstitutionen die Substituenten auch voneinander unabhängig verschieden sein können
oder
- R¹: unter der Voraussetzung, daß R² H ist, Amino bedeutet, welches unsubstituiert ist oder mono- oder di-substituiert ist mit C₁-C₄-Alkyl, Phenyl oder C₁-C₃-Alkylcarbonyl
und
- R²: Wasserstoff oder R¹ bedeutet, wobei R² und R¹ identisch oder verschieden sind
oder
wobei die Reste R¹ und R² zusammen mit dem Stickstoffatom einen Rest der Formel 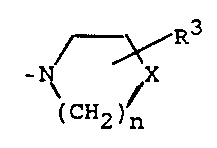 bilden,
worin
- n: 1 bis 3 bedeutet und
- X: O, S, CH₂ oder N-R³ bedeutet,
wobei
- R³: Wasserstoff,Phenyl oder C₁-C₆-Alkyl, C₂-C₆-Alkenyl oder C₂-C₆-Alkinyl bedeutet, wobei diese Phenyl-, Alkyl-, Alkenyl- und Alkinylreste unsubstituiert sind oder ein- oder mehrfach substituiert sind mit:
Phenyl, welches seinerseits unsubstituiert ist oder ein- oder mehrfach substituiert ist mit einem oder mehreren Substituenten, ausgewählt aus: Halogen, Nitro, Cyano, Carboxy, Hydroxy, Methyl, Ethyl, Methoxy, Ethoxy und Trifluormethyl,
oder
N(R⁴)₂, wobei
- R⁴: H oder C₁-C₃-Alkyl bedeutet,
oder
COOR⁵, wobei
- R⁵: H oder C₁-C₃-Alkyl bedeutet,
oder
CON(R⁶)₂ oder CONHR⁶, wobei
- R⁶: H oder C₁-C₃-Alkyl bedeutet, oder wobei (R⁶)₂ eine C₄-C₆-Alkylenkette darstellt, worin keine oder eine CH₂-Gruppe, welche nicht direkt benachbart zu dem Stickstoffatom steht, ersetzt ist durch O, S oder N-R⁴
oder wobei
- R³: C₁-C₄-Alkoxy-carbonyl oder C₃-C₇-Cycloalkyl bedeutet
sowie die physiologisch verträglichen Salze, ebenfalls die Lysin- und Prolin-hydroxylase im Tiermodell wirksam inhibieren.

Dieses Ergebnis ist besonders deshalb überraschend, weil offensichtlich die pharmakologische Aktivität von Pyridinen und Pyridinderivaten nicht ohne weiteres auch auf die entsprechenden Pyrimidine und Pyrimidinderivate übertragen werden kann. So ist beispielsweise das entsprechende lipidsenkende Pyrimidinanaloge der Nicotinsäure nicht bekannt. Gleiches trifft für das Pyrimidinanaloge des Isoniazids zu, welches als Tuberkulosemittel eingesetzt wird.

Im Besonderen betrifft die Erfindung Pyrimidin-4,6-dicarbonsäurediamide gemäß Formel I, worin
- R¹: C₁-C₁₂-Alkyl bedeutet, welches unsubstituiert oder einfach oder im Falle der C₂-C₁₂-Alkyle auch mehrfach substituiert ist mit
Phenyl, Hydroxy, Alkoxy, Amino, Alkoxycarbonyl, Alkyl- oder Dialkylamino, wobei die Alkylreste 1-3 C-Atome aufweisen,
oder
- R¹: Phenyl bedeutet, welches unsubstituiert oder seinerseits mit Halogen, Nitro, Cyano, Methyl oder Methoxy 1-fach substituiert ist,
oder
- R¹: unter der Voraussetzung, daß R² H ist, Amino bedeutet, welches unsubstituiert ist oder mono-substituiert ist mit C₁-C₃-Alkyl, Phenyl oder C₁-C₃-Alkylcarbonyl
und
- R²: Wasserstoff bedeutet,
oder
wobei die Reste R¹ und R² zusammen mit dem Stickstoffatom einen Rest der Formel 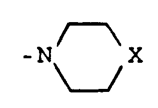 bilden,
worin
- X: O, CH₂ oder N-R³ bedeutet,
wobei
- R³: Wasserstoff, oder C₁-C₃-Alkyl, bedeutet, sowie die physiologisch verträglichen Salze.

Unter Halogen werden Fluor, Chlor, Brom und Jod, unter Aryl Phenyl und Naphthyl und unter Heteroaryl 5- und 6-gliedrige aromatische Ringe mit 1, 2 oder 3 Stickstoff- und/oder Sauerstoff- und/oder Schwefelatomen verstanden, die gegebenenfalls noch benzoanneliert sein können; insbesondere handelt es sich bei den Heteroarylresten um Pyridyl-, Pyridazyl-, Pyrimidyl-, Pyrazyl-, 1,3,5-Triazyl-, Pyrolyl-, Pyrazolyl-, Imidazolyl-, Triazolyl-, Thienyl-, Oxazolyl und Thiazolyl-Reste und gegebenenfalls deren benzoannelierte Verbindungen.

"Mehrfach substituiert" bedeutet im Vorstehenden und Folgenden, daß mindetens 2, höchstens alle in den Alkyl-, Alkenyl-, Alkinyl-, Heteroaryl- und Arylresten vorhandenen Wasserstoffatome durch die genannten Substituenten ersetzt sind. Bei Mehrfachsubstitutionen können die Substituenten auch voneinander unabhängig verschieden sein.

Alle genannten Alkyl- und Alkenylreste mit mehr als 2-C-Atomen und alle Alkinylreste mit mehr als 3-C-Atomen können sowohl geradkettig als auch verzweigt sein.

Weiterhin betrifft die Erfindung die Verbindungen der Formel I zur Anwendung als Arzneimittel. Außerdem betrifft die Erfindung die Verbindungen der Formel I sowie Pyrimidin-4,6-dicarbonsäurediamid (R¹ = R² = H) zur Anwendung als Fibrosuppressiva und Immunsuppressiva sowie zur Inhibierung der Prolin- und Lysinhydroxylase und zur Beeinflussung des Stoffwechsels von Kollagen und kollagenähnlichen Stoffen bzw. der Biosynthese von C1q.

Weiterhin betrifft die Erfindung ein Verfahren zur Herstellung von Verbindungen der Formel I, das dadurch gekennzeichnet ist, daß man eine Verbindung der Formel II 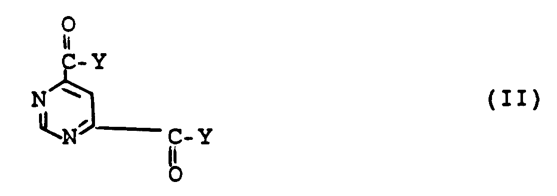
mit einer Verbindung der Formel III, IV oder V 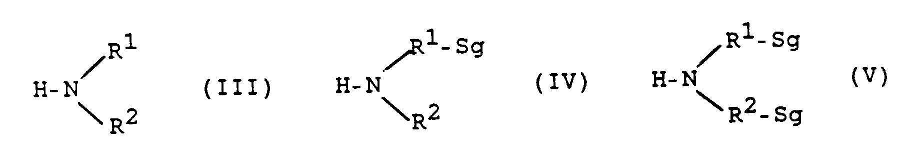
umsetzt, wobei R¹ und R² die zu Formel I angegebenen Bedeutungen haben und Y Halogen, Hydroxy oder C₁-C₄-Alkoxy ist oder zusammen mit der Carbonylgruppe einen Aktivester oder ein gemischtes Anhydrid bildet und Sg eine Schutzgruppe ist
und anschließend in der Verbindung der Formel I die ggf. vorhandene Schutzgruppe entfernt und die Reaktionsprodukte gegebenenfalls in ihre physiologisch verträglichen Salze überführt.

Im Folgenden wird die Herstellung von Verbindungen gemäß Formel I und die Herstellung der dafür benötigten Ausgangssubstanzen - sofern sie nicht käuflich sind - näher beschrieben.

Die Herstellung der erfindungsgemäßen Verbindungen gelingt am einfachsten dadurch, daß die beiden Komponenten, das Pyrimidin-Derivat gemäß Formel (II) und das Amin gemäß Formel (III), (IV) oder (V) in äquimolaren Mengen oder bis zu einem etwa 5-fachen Überschuß an III, IV oder V zusammengegeben werden und bei Temperaturen zwischen -30 bis 150 °C, bevorzugt bei 20 bis 100 °C bis zur Beendigung der Reaktion umgesetzt werden. Die Beendigung der Reaktion läßt sich beispielsweise mittels Dünnschichtchromatographie bestimmen. Eine Variante dieses Verfahrens besteht darin, daß man in einem geeigneten Lösungsmittel, wie Diäthyläther, Dimethoxyäthan oder Tetrahydrofuran, chlorierten Kohlenwasserstoffen wie Methylenchlorid, Chloroform, Tri- oder Tetrachloräthylen, Benzol, Toluol oder auch polaren Lösungsmitteln wie Dimethylformamid, Aceton, Alkoholen wie Methanol oder Ethanol oder Dimethylsulfoxid arbeitet. Auch hier kann ein Überschuß von Amin gemäß Formel (III), (IV) oder (V), der bis zur etwa 5-fachen Mengen betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130°C besonders bevorzugt sind.

Ebenso kann die Umsetzung über ein gemisches Anhydrid wie Chlorameisensäureäthylester oder über einen aktivierten Ester wie Paranitrophenylester (Y= ClCH₂-COO oder NO₂-C₆H₄-O) erfolgen. Entsprechende Methoden sind in der Literatur beschrieben.

Gegebenenfalls kann die Umsetzung auch in Gegenwart von Basen erfolgen. Als zusätzliche Basen kommen beispielsweise Carbonate oder Hydrogencarbonate wie Natrium- oder Kaliumcarbonat oder Natrium- oder Kaliumhydrogencarbonat, oder tertiäre Amine, wie Triäthylamin, Tributylamin, Äthyldiisopropylamin oder heterocyclische Amine wie N-Alkylmorpholin, Pyridin, Chinolin oder Dialkylaniline in Betracht.

Erfolgt die Umsetzung von Verbindungen der Formel II mit Aminen der Formel IV oder V, so erfolgt anschließend die Abspaltung der Schutzgruppe Sg unter den für die gewählte Schutzgruppe geeigneten Bedingungen, die in der Literatur beschrieben sind. Auf diese Weise lassen sich solche Verbindungen der Formel I herstellen, die in den Substituenten R¹ und/oder R² beispielsweise freie OH-, NH₂- oder COOH-Gruppen aufweisen. So geht man beispielsweise zur Herstellung der N,N'-Bis(hydroxyalkyl)-pyrimidin-4,6-dicarbonsäurediamide am besten so vor, daß man ein entsprechendes Bis(alkoxyalkyl)diamid, bevorzugt Bis(methoxyalkyl)diamid, nach literaturbekannten Verfahren, beispielsweise mit Bortribromid, in das entsprechende Bis(hydroxyalkyl)diamid überführt.

Gegebenenfalls kann die Aufarbeitung der Produkte beispielsweise durch Extraktion oder durch Chromatographie z.B. über Kieselgel erfolgen. Das isolierte Produkte kann umkristallisiert und gegebenenfalls mit einer geeigneten Säure zu einem physiologisch verträglichen Salz umgesetzt werden. Als geeignete Säuren kommen beispielsweise in Betracht:
Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion-, Bernstein-, Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (III) können, soweit sie nicht käuflich sind, einfach synthetisiert werden (z.B. Organikum, Organisch Chemisches Grundpraktikum, 15. Aufl., VEB Deutscher Verlag der Wissenschaften, 1976; eine Übersicht über die verschiedenen Möglichkeiten findet sich im Methodenregister, S. 822). Die Amine der Formeln IV und V erhält man, soweit sie nicht käuflich sind, nach literaturbekannten Verfahren aus den ungeschützten Verbindungen durch Umsetzung mit einer Schutzgruppe Sg (Amino- und Carboxyschutzgruppen: Kontakte Merck, 3/79, S. 15 ff; Carboxyschutzgruppen: Houben-Weyl, Methoden der organischen Chemie, Band E5, S. 496-504, 1985; Hydroxyschutzgruppen: Houben-Weyl, Methoden der organischen Chemie, Band VI/1b Alkohole III, S. 735-783, 4. Auflage, Georg Thieme Verlag Stuttgart, New York 1984). Als Aminoschutzgruppen eignen sich beispielsweise Pyoc, Fmoc, Tcboc, Z, Boc, Ddz, Dobz oder Moc. Als Carboxy- und/oder Hydroxyschutzgruppen eignen sich beispielsweise OMe, OBzl, ONbzl, OMbzl, OPic Bu^{t} oder Pac.

Die Ausgangsverbindungen der Formel (II) erhält man beispielsweise durch Umsetzung von Pyrimidin-4,6-dicarbonsäure zu dem entsprechenden Pyrimidin-4,6-dicarbonsäurehalogenid, bevorzugt -chlorid (nach literaturbekannten Verfahren), vorzugsweise in Gegenwart eines Katalysators wie Dimethylformamid. Dieses Säurehalogenid kann dann beispielsweise entweder mit einem geeigneten Alkohol, z.B. Paranitrobenzylalkohol zu dem entsprechenden Aktivester, oder aber mit niederen Alkoholen wie Methanol oder Ethanol zu den entsprechenden Estern umgesetzt werden. Ebenso kann die Pyrimidin-4,6-dicarbonsäure auch zunächst unter Zusatz einer geeigneten Carbonsäure oder eines Carbonsäureesters wie Chlorameisensäureäthylester in ein gemischtes Anhydrid überführt werden, welches dann mit den Aminen (III), (IV) oder (V) zu den erfindungsgemäßen Produkten umgesetzt wird. Eine entsprechende Methode ist ebenfalls in der Literatur beschrieben.

Die Herstellung der Pyrimidin-4,6-dicarbonsäure erfolgt nach literaturbekannten Verfahren, beispielsweise durch Oxidation von 4,6-Dimethylpyrimidin, welches seinerseits beispielsweise erhältlich ist durch katalytische Hydrierung von käuflich erhältlichem 2-Mercapto-4,6-dimethylpyrimidin.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum und Immunsuppressivum.

Auf Grund dieser pharmakologischen Eigenschaften sind die erfindungsgemäßen Verbindungen zur Behandlung von Störungen des Stoffwechsels von Kollagen und kollagenähnlichen Stoffen, bzw. zur Behandlung von Störungen der Biosynthese von C1q geeignet.

Die Erfindung betrifft daher weiter die Anwendung der erfindungsgemäßen Verbindungen der Formel I sowie deren physiologisch verträglichen Salze bei der Behandlung der oben genannten Stoffwechselstörungen.

Die Verbindungen können entweder allein oder mit physiologisch verträglichen Hilfs- oder Trägerstoffen vermischt als Arzneimittel angewandt werden. Sie können zu diesem Zweck oral in Dosen von 0,01 - 25,0 mg/kg/Tag, vorzugsweise 0,01 - 5,0 mg/kg/Tag oder parenteral in Dosen von 0,001 - 5 mg/kg/Tag, vorzugsweise 0,001 - 2,5 mg/kg/Tag, inbesondere 0,005 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral oder rektal appliziert werden.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glukose- oder Mannitlösungen, oder auch eine Mischung aus den verschiedenen genannten Lösungsmitteln.

Nachfolgend ist die Erfindung an Hand von Beispielen näher erläutert.

### Beispiel 1:

### Pyrimidin-4,6-dicarbonsäure-di-(2-methoxyethyl)-amid (Formel I: R¹ = CH₂-CH₂-OCH₃; R² = H)

1,7 g Pyrimidin-4,6-dicarbonsäure werden in 20 ml Toluol suspendiert und 2,4 g Thionylchlorid und 0,2 ml Dimethylformamid zugegeben. Der Ansatz wird zum Rückfluß erhitzt, bis keine Gasentwicklung mehr zu beobachten ist (ca. 3 Stunden). Etwa 5 ml Lösungsmittel werden abdestilliert, der Ansatz auf 0°-10°C abgekühlt und mit 1,9 g 2-Methoxyethylamin und 2,8 ml Triethylamin, gelöst in 10 ml Toluol, versetzt. Die Lösung wird langsam auf Raumtemperatur erwärmt, 12 Stunden bei Raumtemperatur gerührt und zur Trockene eingedampft. Der Rückstand wird mit 50 ml Methylenchlorid aufgenommen, 3 mal mit gesättigter Natriumhydrogencarbonat-Lösung ausgeschüttelt, die organische Phase mit Wasser gewaschen, mit Magnesiumsulfat getrocknet und eingedampft.

Der Festkörper wird aus Diisopropylether umkristallisiert. Ausbeute: 2,1 g ; Fp.: 85-86°C

### Beispiel 2:

### Pyrimidin-4,6-dicarbonsäure-dibenzylamid

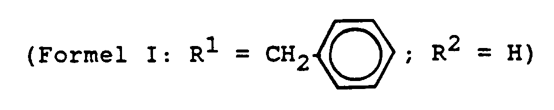

### Versuchsablauf siehe Beispiel 1:

### Ansatz:

1,7 g Pyrimidin-4,6-dicarbonsäure
2,7 g Benzylamin
Ausbeute: 2,1 g ; Fp.: 131-132°C (aus Diisopropylether)

### Beispiel 3:

### Pyrimidin-4,6-dicarbonsäurediethylamid (Formel I: R¹ = CH₂-CH₃; R² = H)

### Versuchsablauf siehe Beispiel 1:

### Ansatz:

1,7 g Pyrimidin-4,6-dicarbonsäure
1,6 g Äthylamin-Hydrochlorid
Ausbeute: 1,1 g ; Fp.: 185-186°C (aus Petrolether)

### Beispiel 4:

### 4,6-Di-[(morpholin-1-yl)-carbonyl]-pyrimidin

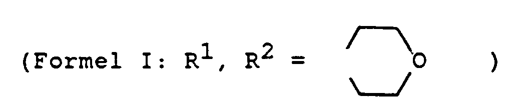

### Versuchsablauf siehe Beispiel 1:

### Ansatz:

1,7 g Pyrimidin-4,6-dicarbonsäure
2,2 g Morpholin
Ausbeute: 2,4 g ; Fp.: 175°C (aus Diisopropylether)

### Beispiel 5:

### Pyrimidin-4,6-dicarbonsäure-di-(3-methoxy-propyl)-amid (Formel I: R¹ = (CH₂)₃-OCH₃; R² = H)

### Versuchsablauf siehe Beispiel 1:

### Ansatz:

8,4 g 4,6-Pyrimidin-dicarbonsäure
11,2 g 3-Methoxypropylamin
Ausbeute: 8,5 g ; Fp.: 64°C (aus Diisopropylether)

### Beispiel 6:

### Pyrimidin-4,6-dicarbonsäure-di-dodecylamid (Formel I: R¹ = (CH₂)₁₁-CH₃; R² = H)

### Versuchsablauf siehe Beispiel 1:

### Ansatz:

0,8 g Pyrimidin-4,6-dicarbonsäure
2,4 g Dodecylamin
Ausbeute: 2,2 g ; Fp.: 78-79°C (aus Diisopropylether)

### Beispiel 7:

### 4,6-Di-[(1-methylpiperazin-4-yl)-carbonyl]-pyrimidin

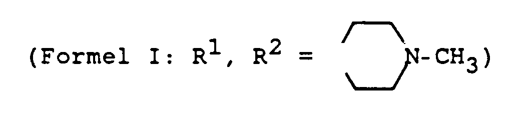

### Versuchsablauf siehe Beispiel 1:

### Ansatz:

0,8 g Pyrimidin-4,6-dicarbonsäure
1,3 g 1-Methylpiperazin
Ausbeute: 1,1 g ; Fp.: 162°C (aus Petrolether)

### Beispiel 8:

### Pyrimidin-4,6-dicarbonsäure-di-(2-diethylamino-ethyl)-amid (Formel I: R¹ = -(CH₂)₂-N(C₂H₅)₂; R² = H)

### Versuchsablauf siehe Beispiel 1:

### Ansatz:

0,8 g Pyrimidin-4,6-dicarbonsäure
1,5 g 2-Diethylamin-ethylamin
Ausbeute: 0,9 g ; Fp.: 72°C (aus Petrolether)

### Beispiel 9:

### Pyrimidin-4,6-dicarbonsäure-di-(2,2-dimethoxy-ethyl)-amid (Formel I: R¹ = CH₂-CH(OCH₃)₂; R² = H)

### Versuchsablauf siehe Beispiel 1:

### Ansatz:

0,8 g Pyrimidin-4,6-dicarbonsäure
1,3 g Aminoacetaldehyd-dimethylacetal
Ausbeute: 1,0 g ; Fp.: 107°C (aus Petrolether)

### Beispiel 10:

### Pyrimidin-4,6-dicarbonsäure-di-anilid

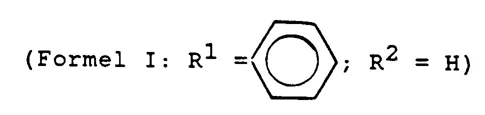

### Versuchsablauf siehe Beispiel 1:

### Ansatz:

0,8 g Pyrimidin-4,6-dicarbonsäure
1,2 g Anilin
Ausbeute: 0,8 g ; Fp.: 225°C (aus Petrolether)

### Beispiel 11:

### Pyrimidin-4,6-dicarbonsäure-di-(2-methoxy-isopropyl)-amid (Formel I: R¹ = CH(CH₂OCH₃)CH₃; R² = H)

### Versuchsablauf siehe Beispiel 1:

### Ansatz:

0,8 g Pyrimidin-4,6-dicarbonsäure
1,1 g 2-Amino-1-methoxypropan
Ausbeute: 1,0 g ; Fp.: 55°C (aus Petrolether)

### Beispiel 12:

### Pyrimidin-4,6-dicarbonsäure-di-(2-hydroxy-ethyl)-amid (Formel I: R¹ = CH₂-CH₂-OH; R² = H)

0,9 g Pyrimidin-4,6-dicarbonsäure-di-(2-methoxy-ethyl)-amid aus Beispiel 1 werden bei Raumtemperatur in 5 ml Methylenchlorid gelöst, auf -78°C abgekühlt und 18 ml Bortribromid (1M-Lösung in Dichlormethan) langsam über 1 Stunde zugetropft. Man läßt auf Raumtemperatur kommen und rührt 3 Stunden nach. Danach wird der Ansatz auf 120 ml Natriumhydrogencarbonat-Lösung gegossen und 3 mal mit Ethylacetat extrahiert. Die vereinigten organischen Lösungsmittel werden mit Magnesiumsulfat getrocknet und eingedampft. Das Rohprodukt wird an Kieselgel chromatographiert.
Ausbeute: 0,8 g ; Fp.: 62°C

### Beispiel 13:

### Pyrimidin-4,6-dicarbonsäure-di-(3-hydroxypropyl)-amid (Formel I: R¹ = (CH₂)₃-OH; R² = H)

Analog Beispiel 12 wird die Verbindung aus Pyrimidin-4,6-dicarbonsäure-di-(3-methoxypropyl)-amid (Beispiel 5) hergestellt.

### Beispiel 14:

### Pyrimidin-4,6-dicarbonsäure-dihydrazid (Formel I: R¹ = -NH₂; R² = H)

2 g Pyrimidin-4,6-dicarbonsäuredimethylester (hergestellt gemäß H. Yamada, Heterocycles, 13, 235 (1979)) werden bei Raumtemperatur in 75 ml Methanol gelöst. Es werden 1,1 g Hydrazinhydrat zugegeben. Es entsteht ein gelber Niederschlag, der 3 Stunden gerührt und dann abgesaugt wird.
Ausbeute: 1,9 g ; Fp.:

### Beispiel 15:

### Pyrimidin-4,6-dicarbonsäure-di-acetohydrazid (Formel I: R¹ = NH-C(O)-CH₃; R² = H)

0,4 g Pyrimidin-4,6-dicarbonsäuredihydrazid aus Beispiel 14 werden in 25 ml Dichlormethan bei Raumtemperatur suspendiert. Es werden 0,2 g 4-Dimethylaminopyridin und 0,4 g Essigsäureanhydrid zugegeben und 12 Stunden bei Raumtemperatur gerührt. Es wird zur Trockne eingeengt, mit Ethylacetat:Cyclohexan 4:1 ausgerührt, der entstandene Rückstand abgesaugt und getrocknet.
Ausbeute: 0,33 g ; Fp.:

### Beispiel 16:

### Pharmakologische Wirksamkeit

Zum Nachweis der effektiven Hemmung der Prolin- und Lysinhydroxylase durch die erfindungsgemäßen Verbindungen wurden die Hydroxyprolin-Konzentrationen in der Leber und die Prokollagen-III-peptid und Bilirubin-Konzentrationen im Serum von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindunggemäße Verbindung verabreicht wurde
gemessen (diese Testmethode wird beschrieben von Rouiller, C., experimental toxic injury of the liver; in The Liver, C. Rouiller, Vol. 2, S. 335-476, New York, Academic Press, 1964).

Die Wirkstärke der erfindungsgemäßen Verbindungen wurde als prozentuale Hemmung der Leber-Hydroxyprolin- und Prokollagen-III-peptid und Bilirubinsynthese nachoraler Gabe im Vergleich zu Kontrolltieren, denen nur Tetrachlorkohlenstoff verabreicht wurde (CCl₄-Kontrolle), bestimmt. Die Ergebnisse sind in Tabelle 1 aufgeführt.

**Tabelle 1**

| Wirkung von Prolylhydroxylase inhibitoren auf durch CCl₄ induzierte Leberfibrose in Ratten, 8-Wochen-Behandlung | | | | | |
|---|---|---|---|---|---|
| Verbindung | Dosis mg/kg | N | Bilirubin^{b} »m | PIIIP^{c} ng/ml | Hyp^{d} »g/ml |
| Kontrolle | | 20 | 1.9 | 11 | 0.056 |
| CCl₄ | | 56 | 4.5 | 32 | 0.228 |
| Zunahme^{e} | | 36 | 2.6 | 21 | 0.172 |
| Beispiel 3 | 50 | 17 | 2.8 | 26 | 0.200 |
| Zunahme^{e} | | | 0.9 | 15 | 0.144 |
| Abnahme^{f} | | | 65 | 28 | 16 % |
| Mittelwert^{g} | | | (36 ± 15 %) | | |
| Beispiel 2 | 50 | 16 | 2.8 | 26 | 0.192 |
| Zunahme^{e} | | | 0.9 | 28 | 0.136 |
| Abnahme^{f} | | | 65 | 28 | 21 % |
| Mittelwert^{g} | | | (38 ± 14 %) | | |

| | | | | | |
|---|---|---|---|---|---|
| ^{a}: tägliche orale Dosis | | | | | |
| ^{b}: Bilirubin im Serum (total) | | | | | |
| ^{c}: Prokollagen III N-Peptid im Serum | | | | | |
| ^{d}: Hydroxyprolin-Gehalt in der Leber | | | | | |
| ^{e}: Zunahme (absolut) gegen Kontrolle | | | | | |
| ^{f}: prozentuale Abnahme gegen CCl₄-Behandlung | | | | | |
| ^{g}: Gesamtgehalt an Bilirubin, PIIIP und Hyp, % Abweichung | | | | | |

## Patentansprüche (Patentansprüche für folgende(n) Vertragsstaat(en): AT, BE, CH, DE, DK, FR, GB, GR, IT, LI, NL, SE)

1. Pyrimidin-4,6-dicarbonsäurediamide der Formel I 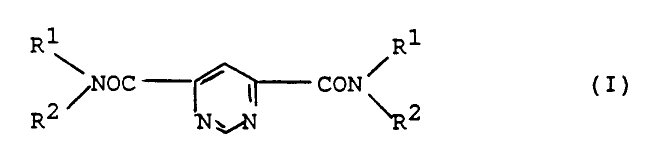 worin
R¹ C₁-C₁₂-Alkyl, C₂-C₁₂-Alkenyl oder C₂-C₁₂-Alkinyl bedeutet, welche unsubstituiert oder einfach oder im Falle der C₂-C₁₂-Alkyle, C₂-C₁₂-Alkenyle und C₂-C₁₂-Alkinyle auch mehrfach substituiert sind mit
Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, oder mit
Indolyl oder Phenyl, welches unsubstituiert ist oder mit Halogen, Nitro, C₁-C₄-Alkyl oder C₁-C₄-Alkoxy 1-, 2-, oder 3-fach substituiert ist, wobei bei Mehrfachsubstitutionen die Substituenten auch voneinander unabhängig verschieden sein können
oder
R¹ gesättigtes C₅-C₇-Cycloalkyl bedeutet, welches gegebenenfalls benzoanneliert ist,
oder
R¹ Aryl oder Heteroaryl bedeutet, welches unsubstituiert oder seinerseits mit Halogen, Nitro, Cyano, C₁-C₄-Alkyl oder C₁-C₄-Alkoxy 1-, 2-, oder 3-fach substituiert ist, wobei bei Mehrfachsubstitutionen die Substituenten auch voneinander unabhängig verschieden sein können
oder
R¹ unter der Voraussetzung, daß R² H ist, Amino bedeutet, welches unsubstituiert ist oder mono- oder di-substituiert ist mit C₁-C₄-Alkyl, Phenyl oder C₁-C₃-Alkylcarbonyl
und
R² Wasserstoff oder R¹ bedeutet, wobei R² und R¹ identisch oder verschieden sind
oder
wobei die Reste R¹ und R² zusammen mit dem Stickstoffatom einen Rest der Formel 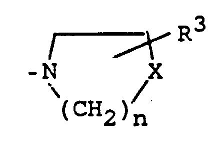 bilden,
worin
n 1 bis 3 bedeutet und
X 0, S, CH₂ oder N-R³ bedeutet,
wobei
R³ Wasserstoff,Phenyl oder C₁-C₆-Alkyl, C₂-C₆-Alkenyl oder C₂-C₆-Alkinyl bedeutet, wobei diese Phenyl-, Alkyl-, Alkenyl- und Alkinylreste unsubstituiert sind oder ein- oder mehrfach substituiert sind mit:
Phenyl, welches seinerseits unsubstituiert ist oder ein- oder mehrfach substituiert ist mit einem oder mehreren Substituenten, ausgewählt aus: Halogen, Nitro, Cyano, Carboxy, Hydroxy, Methyl, Ethyl, Methoxy, Ethoxy und Trifluormethyl,
oder
N(R⁴)₂, wobei
R⁴ H oder C₁-C₃-Alkyl bedeutet,
oder
COOR⁵, wobei
R⁵ H oder C₁-C₃-Alkyl bedeutet,
oder
CON(R⁶)₂ oder CONHR⁶ wobei
R⁶ H oder C₁-C₃-Alkyl bedeutet, oder wobei (R⁶)₂ eine C₄-C₆-Alkylenkette darstellt, worin keine oder eine CH₂-Gruppe, welche nicht direkt benachbart zu dem Stickstoffatom steht, ersetzt ist durch 0, S oder N-R⁴
oder wobei
R³ C₁-C₄-Alkoxy-carbonyl oder C₃-C₇-Cycloalkyl bedeutet
sowie die physiologisch verträglichen Salze.

2. Pyrimidin-4,6-dicarbonsäurediamide der Formel I nach Anspruch 1, worin
R¹ C₁-C₁₂-Alkyl bedeutet, welches unsubstituiert oder einfach oder im Falle der C₂-C₁₂-Alkyle auch mehrfach substituiert ist mit
Phenyl, Hydroxy, Alkoxy, Amino, Alkoxycarbonyl, Alkyl- oder Dialkylamino, wobei die Alkylreste 1-3 C-Atome aufweisen,
oder
R¹ Phenyl bedeutet, welches unsubstituiert oder seinerseits mit Halogen, Nitro, Cyano, Methyl oder Methoxy 1-fach substituiert ist,
oder
R¹ unter der Voraussetzung, daß R² H ist, Amino bedeutet, welches unsubstituiert ist oder mono-substituiert ist mit C₁-C₃-Alkyl, Phenyl oder C₁-C₃-Alkylcarbonyl
und
R² Wasserstoff bedeutet,
oder
wobei die Reste R¹ und R² zusammen mit dem Stickstoffatom einen Rest der Formel 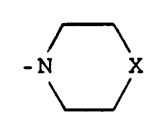 bilden,
worin
X 0, CH₂ oder N-R³ bedeutet,
wobei
R³ Wasserstoff, oder C₁-C₃-Alkyl, bedeutet, sowie die physiologisch verträglichen Salze.

3. Pyrimidin-4,6-dicarbonsäurediamide der Formel I nach Anspruch 1, worin
R¹ C₁-C₁₂-Alkyl, bedeutet, welches unsubstituiert oder einfach oder im Falle der C₂-C₁₂-Alkyle auch mehrfach substituiert ist mit
Phenyl, Hydroxy, Alkoxy, Alkoxycarbonyl, oder Dialkylamino, wobei die Alkylreste 1-3 C-Atome aufweisen,
oder
R¹ Phenyl bedeutet,
oder
R¹ unter der Voraussetzung, daß R² H ist, Amino bedeutet, welches unsubstituiert ist/oder mono-substituiert ist mit Methylcarbonyl
und
R² Wasserstoff bedeutet,
oder
wobei die Reste R¹ und R² zusammen mit dem Stickstoffatom einen Rest der Formel 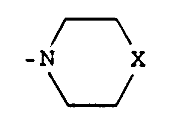 bilden,
worin
X 0, CH₂ oder N-R³ bedeutet,
wobei
R³ Wasserstoff oder Methyl bedeutet, sowie die physiologisch verträglichen Salze.

4. Verfahren zur Herstellung von Verbindungen der Formel I, dadurch gekennzeichnet, daß man eine Verbindung der Formel II 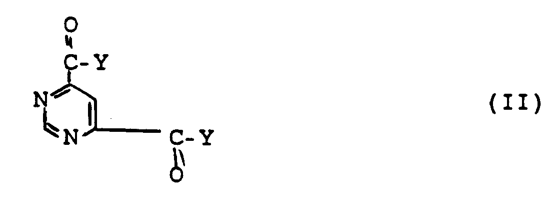 mit einer Verbindung der Formel III, IV oder V 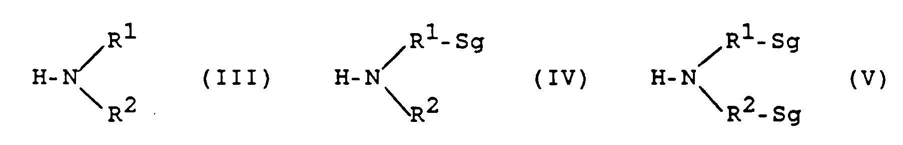 umsetzt, wobei R¹ und R² die zu Formel I angegebenen Bedeutungen haben und Y Halogen, Hydroxy oder C₁-C₄-Alkoxy ist oder zusammen mit der Carbonylgruppe einen Aktivester oder ein gemischtes Anhydrid bildet und Sg eine Schutzgruppe ist
und anschließend in der Verbindung der Formel I die ggf. vorhandenen Schutzgruppen entfernt und die Reaktionsprodukte gegebenenfalls in ihre physiologisch verträglichen Salze überführt.

5. Verbindungen nach einem oder mehreren der Ansprüche 1 bis 3 zur Anwendung als Arzneimittel.

6. Verbindungen nach einem oder mehreren der Ansprüche 1 bis 3 sowie Pyrimidin-4,6-dicarbonsäurediamid zur Inhibierung der Prolin- und Lysinhydroxylase.

7. Verbindungen nach einem oder mehreren der Ansprüche 1 bis 3 sowie Pyrimidin-4,6-dicarbonsäurediamid zur Anwendung als Fibrosuppressiva und Immunsuppressiva.

8. Arzneimittel, enthaltend eine Verbindung der Formel I mit verträglichen pharmazeutischen Trägern.

9. Verwendung von Verbindungen der Formel I sowie Pyrimidin-4,6-dicarbonsäurediamid zur Herstellung eines Arzneimittels zur Beeinflussung des Stoffwechsels von Kollagen und kollagenähnlichen Stoffen bzw. der Biosynthese von C1_{q}.

10. Verwendung von Verbindungen der Formel I sowie Pyrimidin-4,6-dicarbonsäurediamid zur Herstellung eines Arzneimittels zur Behandlung von Störungen des Stoffwechsels von Kollagen und kollagenähnlichen Stoffen bzw. der Biosynthese von C1_{q}.

11. Verfahren zur Herstellung von Arzneimitteln zur Beeinflussung des Stoffwechsels von Kollagen und kollagenähnlichen Stoffen bzw. der Biosynthese von C1_{q}, dadurch gekennzeichnet, daß man in das Arzneimittel eine Verbindung der Formel I und/oder Pyrimidin-4,6-dicarbonsäurediamid einverleibt.

## Patentansprüche (Patentansprüche für folgende(n) Vertragsstaat(en): ES)

1. Verfahren zur Herstellung von Verbindungen der Formel I 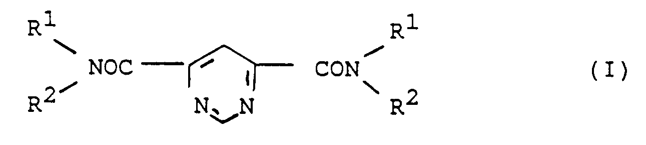 worin
R¹ C₁-C₁₂-Alkyl, C₂-C₁₂-Alkenyl oder C₂-C₁₂-Alkinyl bedeutet, welche unsubstituiert oder einfach oder im Falle der C₂-C₁₂-Alkyle, C₂-C₁₂-Alkenyle und C₂-C₁₂-Alkinyle auch mehrfach substituiert sind mit
Halogen, Hydroxy, Cyano, Amino, Carboxyl, Alkoxy, Alkoxycarbonyl, Alkylcarbonyloxy, Alkyl- oder Dialkylamino, wobei die Alkylreste 1-4 C-Atome aufweisen, oder mit
Indolyl oder Phenyl, welches unsubstituiert ist oder mit Halogen, Nitro, C₁-C₄-Alkyl oder C₁-C₄-Alkoxy 1-, 2-, oder 3-fach substituiert ist, wobei bei Mehrfachsubstitutionen die Substituenten auch voneinander unabhängig verschieden sein können
oder
R¹ gesättigtes C₅-C₇-Cycloalkyl bedeutet, welches gegebenenfalls benzoanneliert ist,
oder
R¹ Aryl oder Heteroaryl bedeutet, welches unsubstituiert oder seinerseits mit Halogen, Nitro, Cyano, C₁-C₄-Alkyl oder C₁-C₄-Alkoxy 1-, 2-, oder 3-fach substituiert ist, wobei bei Mehrfachsubstitutionen die Substituenten auch voneinander unabhängig verschieden sein können
oder
R¹ unter der Voraussetzung, daß R² H ist, Amino bedeutet, welches unsubstituiert ist oder mono- oder di-substituiert ist mit C₁-C₄-Alkyl, Phenyl oder C₁-C₃-Alkylcarbonyl
und
R² Wasserstoff oder R¹ bedeutet, wobei R² und R¹ identisch oder verschieden sind
oder
wobei die Reste R¹ und R² zusammen mit dem Stickstoffatom einen Rest der Formel 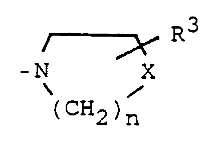 bilden,
worin
n 1 bis 3 bedeutet und
X 0, S, CH₂ oder N-R³ bedeutet,
wobei
R³ Wasserstoff,Phenyl oder C₁-C₆-Alkyl, C₂-C₆-Alkenyl oder C₂-C₆-Alkinyl bedeutet, wobei diese Phenyl-, Alkyl-, Alkenyl und Alkinylreste unsubstituiert sind oder ein- oder mehrfach substituiert sind mit:
Phenyl, welches seinerseits unsubstituiert ist oder ein- oder mehrfach substituiert ist mit einem oder mehreren Substituenten, ausgewählt aus: Halogen, Nitro, Cyano, Carboxy, Hydroxy, Methyl, Ethyl, Methoxy, Ethoxy und Trifluormethyl,
oder
N(R⁴)₂, wobei
R⁴ H oder C₁-C₃-Alkyl bedeutet,
oder
COOR⁵, wobei
R⁵ H oder C₁-C₃-Alkyl bedeutet,
oder
CON(R⁶)₂ oder CONHR⁶ wobei
R⁶ H oder C₁-C₃-Alkyl bedeutet, oder wobei (R⁶)₂ eine C₄-C₆-Alkylenkette darstellt, worin keine oder eine CH₂-Gruppe, welche nicht direkt benachbart zu dem Stickstoffatom steht, ersetzt ist durch 0, S oder N-R⁴
oder wobei
R³ C₁-C₄-Alkoxy-carbonyl oder C₃-C₇-Cycloalkyl bedeutet
sowie die physiologisch verträglichen Salze, dadurch gekennzeichnet, daß man eine Verbindung der Formel II 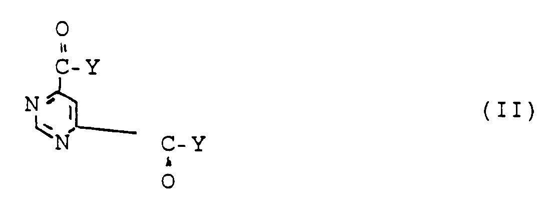 mit einer Verbindung der Formel III, IV oder V 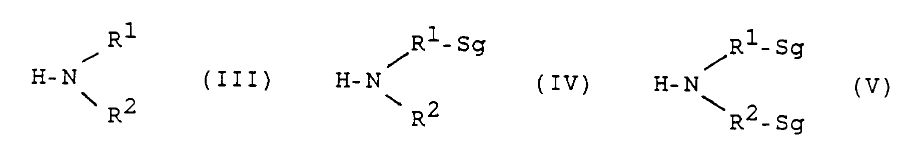 umsetzt, wobei R¹ und R² die zu Formel I angegebenen Bedeutungen haben und Y Halogen, Hydroxy oder C₁-C₄-Alkoxy ist oder zusammen mit der Carbonylgruppe einen Aktivester oder ein gemischtes Anhydrid bildet und Sg eine Schutzgruppe ist
und anschließend in der Verbindung der Formel I die ggf. vorhandenen Schutzgruppen entfernt und die Reaktionsprodukte gegebenenfalls in ihre physiologisch verträglichen Salze überführt.

2. Verfahren nach Anspruch 1, worin
R¹ C₁-C₁₂-Alkyl bedeutet, welches unsubstituiert oder einfach oder im Falle der C₂-C₁₂-Alkyle auch mehrfach substituiert ist mit
Phenyl, Hydroxy, Alkoxy, Amino, Alkoxycarbonyl, Alkyl- oder Dialkylamino, wobei die Alkylreste 1-3 C-Atome aufweisen,
oder
R¹ Phenyl bedeutet, welches unsubstituiert oder seinerseits mit Halogen, Nitro, Cyano, Methyl oder Methoxy 1-fach substituiert ist,
oder
R¹ unter der Voraussetzung, daß R² H ist, Amino bedeutet, welches unsubstituiert ist oder mono-substituiert ist mit C₁-C₃-Alkyl, Phenyl oder C₁-C₃-Alkylcarbonyl
und
R² Wasserstoff bedeutet,
oder
wobei die Reste R¹ und R² zusammen mit dem Stickstoffatom einen Rest der Formel 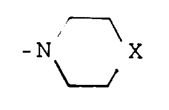 bilden,
worin
X 0, CH₂ oder N-R³ bedeutet,
wobei
R³ Wasserstoff, oder C₁-C₃-Alkyl, bedeutet, sowie die physiologisch verträglichen Salze.

3. Verfahren nach Anspruch 1, worin
R¹ C₁-C₁₂-Alkyl, bedeutet, welches unsubstituiert oder einfach oder im Falle der C₂-C₁₂-Alkyle auch mehrfach substituiert ist mit
Phenyl, Hydroxy, Alkoxy, Alkoxycarbonyl, oder Dialkylamino, wobei die Alkylreste 1-3 C-Atome aufweisen,
oder
R¹ Phenyl bedeutet,
oder
R¹ unter der Voraussetzung, daß R² H ist, Amino bedeutet, welches unsubstituiert ist oder mono-substituiert ist mit Methylcarbonyl
und
R² Wasserstoff bedeutet,
oder
wobei die Reste R¹ und R² zusammen mit dem Stickstoffatom einen Rest der Formel 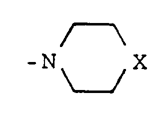 bilden,
worin
X 0, CH₂ oder N-R³ bedeutet,
wobei
R³ Wasserstoff oder Methyl bedeutet, sowie die physiologisch verträglichen Salze.

4. Verbindungen nach einem oder mehreren der Ansprüche 1 bis 3 zur Anwendung als Arzneimittel.

5. Verbindungen nach einem oder mehreren der Ansprüche 1 bis 3 sowie Pyrimidin-4,6-dicarbonsäurediamid zur Inhibierung der Prolin- und Lysinhydroxylase.

6. Verbindungen nach einem oder mehreren der Ansprüche 1 bis 3 sowie Pyrimidin-4,6-dicarbonsäurediamid zur Anwendung als Fibrosuppressiva und Immunsuppressiva.

7. Arzneimittel, enthaltend eine Verbindung der Formel I mit verträglichen pharmazeutischen Trägern.

8. Verwendung von Verbindungen der Formel I sowie Pyrimidin-4,6-dicarbonsäurediamid zur Herstellung eines Arzneimittels zur Beeinflussung des Stoffwechsels von Kollagen und kollagenähnlichen Stoffen bzw. der Biosynthese von Clq.

9. Verwendung von Verbindungen der Formel I sowie Pyrimidin-4,6-dicarbonsäurediamid zur Herstellung eines Arzneimittels zur Behandlung von Störungen des Stoffwechsels von Kollagen und kollagenähnlichen Stoffen bzw. der Biosynthese von C1q.

10. Verfahren zur Herstellung von Arzneimitteln zur Beeinflussung des Stoffwechsels von Kollagen und kollagenähnlichen Stoffen bzw. der Biosynthese von C1q, dadurch gekennzeichnet, daß man in das Arzneimittel eine Verbindung der Formel I und/oder Pyrimidin-4,6-dicarbonsäurediamid einverleibt.

## Claims (Claims for the following Contracting State(s): AT, BE, CH, DE, DK, FR, GB, GR, IT, LI, NL, SE)

1. A pyrimidine-4,6-dicarboxylic acid diamide of the formula I 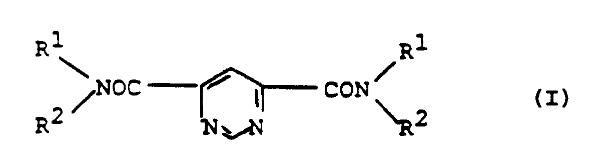 in which
R¹ is C₁-C₁₂-alkyl, C₂-C₁₂-alkenyl or C₂-C₁₂-alkynyl, which are unsubstituted or monosubstituted or alternatively, in the case of C₂-C₁₂-alkyls, C₂-C₁₂-alkenyls and C₂-C₁₂-alkynyls, are polysubstituted by
halogen, hydroxyl, cyano, amino, carboxyl, alkoxy, alkoxycarbonyl, alkylcarbonyloxy or alkyl- or dialkylamino, in which the alkyl radicals contain 1-4 carbon atoms, or by
indolyl or phenyl, which is unsubstituted or substituted by 1, 2 or 3 substituents from the group comprising halogen, nitro, C₁-C₄-alkyl and C₁-C₄-alkoxy, it also being possible for the substituents to differ independently of one another in the case of polysubstitutions,
or
R¹ is saturated C₅-C₇-cycloalkyl, which is optionally benzo-fused,
or
R¹ is aryl or heteroaryl, which is unsubstituted or in turn substituted by 1, 2 or 3 substituents from the group comprising halogen, nitro, cyano, C₁-C₄-alkyl and C₁-C₄-alkoxy, it also being possible for the substituents to differ independently of one another in the case of polysubstitutions,
or
R¹, provided that R² is H, is amino, which is unsubstituted or mono- or disubstituted by C₁-C₄-alkyl, phenyl or C₁-C₃-alkylcarbonyl,
and
R² is hydrogen or R¹, R² and R¹ being identical or different,
or in which the radicals R¹ and R², together with the nitrogen atom, form a radical of the formula 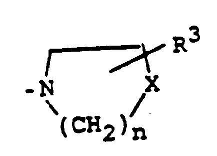 in which
n is 1 to 3 and
X is O, S, CH₂ or N-R³, in which
R³ is hydrogen, phenyl or C₁-C₆-alkyl, C₂-C₆-alkenyl or C₂-C₆-alkynyl, these phenyl, alkyl, alkenyl and alkynyl radicals being unsubstituted or mono- or polysubstituted by:
phenyl, which is in turn unsubstituted or mono- or polysubstituted by one or more substituents chosen from: halogen, nitro, cyano, carboxyl, hydroxyl, methyl, ethyl, methoxy, ethoxy and trifluoromethyl,
or
N(R⁴)₂, in which
R⁴ is H or C₁-C₃-alkyl,
or
COOR⁵, in which
R⁵ is H or C₁-C₃-alkyl,
or
CON(R⁶)₂ or CONHR⁶, in which
R⁶ is H or C₁-C₃-alkyl, or in which (R⁶)₂ represents a C₄-C₆-alkylene chain in which no CH₂ group or a CH₂ group which is not directly adjacent to the nitrogen atom is replaced by O, S or N-R⁴,
or in which
R³ is C₁-C₄-alkoxy-carbonyl or C₃-C₇-cycloalkyl,
or a physiologically tolerated salt.

2. A pyrimidine-4,6-dicarboxylic acid diamide of the formula I as claimed in claim 1, in which
R¹ is C₁-C₁₂-alkyl, which is unsubstituted or monosubstituted or alternatively, in the case of C₂-C₁₂-alkyls, is polysubstituted by
phenyl, hydroxyl, alkoxy, amino, alkoxycarbonyl or alkyl- or dialkylamino, in which the alkyl radicals contain 1-3 carbon atoms,
or
R¹ is phenyl, which is unsubstituted or in turn monosubstituted by halogen, nitro, cyano, methyl or methoxy,
or
R¹, provided that R² is H, is amino, which is unsubstituted or monosubstituted by C₁-C₃-alkyl, phenyl or C₁-C₃-alkylcarbonyl,
and
R² is hydrogen,
or in which the radicals R¹ and R², together with the nitrogen atom, form a radical of the formula 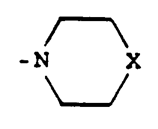 in which
X is O, CH₂ or N-R³,
in which
R³ is hydrogen or C₁-C₃-alkyl, or a physiologically tolerated salt.

3. A pyrimidine-4,6-dicarboxylic acid diamide of the formula I as claimed in claim 1, in which
R¹ is C₁-C₁₂-alkyl, which is unsubstituted or monosubstituted or alternatively, in the case of C₂-C₁₂-alkyls, is polysubstituted by
phenyl, hydroxyl, alkoxy, alkoxycarbonyl or dialkylamino, in which the alkyl radicals contain 1-3 carbon atoms,
or
R¹ is phenyl,
or
R¹, provided that R² is H, is amino, which is unsubstituted or monosubstituted by methylcarbonyl,
and
R² is hydrogen,
or in which the radicals R¹ and R², together with the nitrogen atom, form a radical of the formula 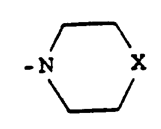 in which
X is O, CH₂ or N-R³,
in which
R³ is hydrogen or methyl, or a physiologically tolerated salt.

4. A process for the preparation of a compound of the formula I, which comprises reacting a compound of the formula II 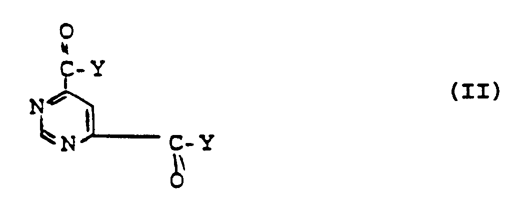 with a compound of the formula III, IV or V 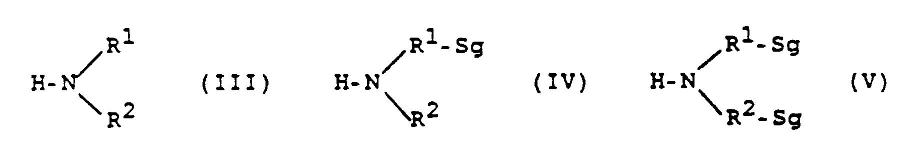 in which R¹ and R² have the meanings given in the case of formula I and Y is halogen, hydroxyl or C₁-C₄-alkoxy or, together with the carbonyl group, forms an active ester or a mixed anhydride, and Sg is a protective group,
and subsequently removing any protective groups present in the compound of the formula I, and if appropriate converting the reaction product into one of its physiologically tolerated salts.

5. A compound as claimed in one or more of claims 1 to 3 for use as a pharmaceutical.

6. A compound as claimed in one or more of claims 1 to 3 and pyrimidine-4,6-dicarboxylic acid diamide for inhibition of proline hydroxylase and lysine hydroxylase.

7. A compound as claimed in one or more of claims 1 to 3 and pyrimidine-4,6-dicarboxylic acid diamide for use as a fibrosuppressant and immunosuppressant.

8. A pharmaceutical containing a compound of the formula I with tolerated pharmaceutical excipients.

9. The use of a compound of the formula I and pyrimidine-4,6-dicarboxylic acid diamide for preparing a pharmaceutical for influencing the metabolism of collagen and collagen-like substances and the biosynthesis of C1_{q}.

10. The use of a compound of the formula I and pyrimidine-4,6-dicarboxylic acid diamide for preparing a pharmaceutical for the treatment of disturbances in the metabolism of collagen and collagen-like substances or the biosynthesis of C1_{q}.

11. A process for the preparation of pharmaceuticals for influencing the metabolism of collagen and collagen-like substances and the biosynthesis of C1_{q}, which comprises incorporating a compound of the formula I and/or pyrimidine-4,6-dicarboxylic acid diamide into the pharmaceutical.

## Claims (Claims for the following Contracting State(s): ES)

1. A process for the preparation of a compound of the formula I 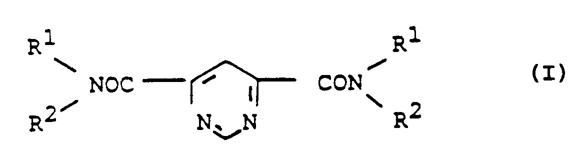 in which
R¹ is C₁-C₁₂-alkyl, C₂-C₁₂-alkenyl or C₂-C₁₂-alkynyl, which are unsubstituted or monosubstituted or alternatively, in the case of C₂-C₁₂-alkyls, C₂-C₁₂-alkenyls and C₂-C₁₂-alkynyls, are polysubstituted by
halogen, hydroxyl, cyano, amino, carboxyl, alkoxy, alkoxycarbonyl, alkylcarbonyloxy or alkyl- or dialkylamino, in which the alkyl radicals contain 1-4 carbon atoms, or by
indolyl or phenyl, which is unsubstituted or substituted by 1, 2 or 3 substituents from the group comprising halogen, nitro, C₁-C₄-alkyl or C₁-C₄-alkoxy, it also being possible for the substituents to differ independently of one another in the case of polysubstitutions,
or
R¹ is saturated C₅-C₇-cycloalkyl, which is optionally benzo-fused,
or
R¹ is aryl or heteroaryl, which is unsubstituted or in turn substituted by 1, 2 or 3 substituents from the group comprising halogen, nitro, cyano, C₁-C₄-alkyl and C₁-C₄-alkoxy, it also being possible for the substituents to differ independently of one another in the case of polysubstitutions,
or
R¹, provided that R² is H, is amino, which is unsubstituted or mono- or disubstituted by C₁-C₄-alkyl, phenyl or C₁-C₃-alkylcarbonyl,
and
R² is hydrogen or R¹, R² and R¹ being identical or different,
or in which the radicals R¹ and R², together with the nitrogen atom, form a radical of the formula 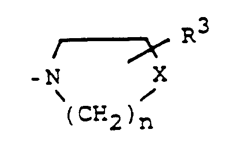 in which
n is 1 to 3 and
X is O, S, CH₂ or N-R³,
in which
R³ is hydrogen, phenyl or C₁-C₆-alkyl, C₂-C₆-alkenyl or C₂-C₆-alkynyl, these phenyl, alkyl, alkenyl and alkynyl radicals being unsubstituted or mono- or polysubstituted by:
phenyl, which is in turn unsubstituted or mono- or polysubstituted by one or more substituents chosen from: halogen, nitro, cyano, carboxyl, hydroxyl, methyl, ethyl, methoxy, ethoxy and trifluoromethyl,
or
N(R⁴)₂, in which
R⁴ is H or C₁-C₃-alkyl,
or
COOR⁵, in which
R⁵ is H or C₁-C₃-alkyl,
or
CON(R⁶)₂ or CONHR⁶, in which
R⁶ is H or C₁-C₃-alkyl, or in which (R⁶)₂ represents a C₄-C₆-alkylene chain in which no CH₂ group or a CH₂ group which is not directly adjacent to the nitrogen atom is replaced by O, S or N-R⁴,
or in which
R³ is C₁-C₄-alkoxy-carbonyl or C₃-C₇-cycloalkyl,
or a physiologically tolerated salt, which comprises reacting a compound of the formula II 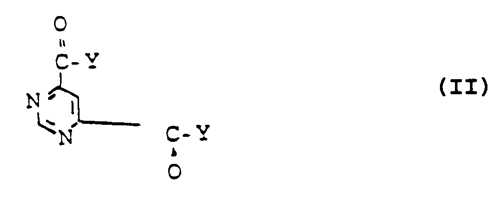 with a compound of the formula III, IV or V 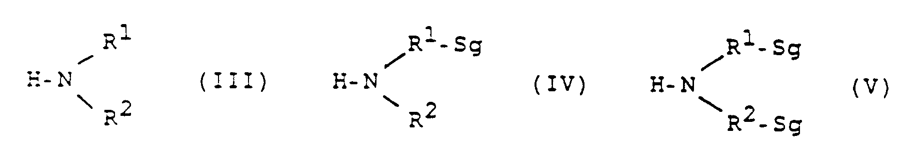 in which R¹ and R² have the meanings given in the case of formula I and Y is halogen, hydroxyl or C₁-C₄-alkoxy or, together with the carbonyl group, forms an active ester or a mixed anhydride, and Sg is a protective group, and subsequently removing any protective groups present in the compound of the formula I, and if appropriate converting the reaction product into one of its physiologically tolerated salts.

2. The process as claimed in claim 1, in which
R¹ is C₁-C₁₂-alkyl, which is unsubstituted or monosubstituted or alternatively, in the case of C₂-C₁₂-alkyls, is polysubstituted by
phenyl, hydroxyl, alkoxy, amino, alkoxycarbonyl or alkyl- or dialkylamino, in which the alkyl radicals contain 1-3 carbon atoms,
or
R¹ is phenyl, which is unsubstituted or in turn monosubstituted by halogen, nitro, cyano, methyl or methoxy,
or
R¹, provided that R² is H, is amino, which is unsubstituted or monosubstituted by C₁-C₃-alkyl, phenyl or C₁-C₃-alkylcarbonyl,
and
R² is hydrogen,
or in which the radicals R¹ and R², together with the nitrogen atom, form a radical of the formula 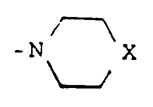 in which
X is O, CH₂ or N-R³,
in which
R³ is hydrogen or C₁-C₃-alkyl, or a physiologically tolerated salt.

3. The process as claimed in claim 1, in which
R¹ is C₁-C₁₂-alkyl, which is unsubstituted or monosubstituted or alternatively, in the case of C₂-C₁₂-alkyls, is polysubstituted by
phenyl, hydroxyl, alkoxy, alkoxycarbonyl or dialkylamino, in which the alkyl radicals contain 1-3 carbon atoms,
or
R¹ is phenyl,
or
R¹, provided that R² is H, is amino, which is unsubstituted or monosubstituted by methylcarbonyl,
and
R² is hydrogen,
or in which the radicals R¹ and R², together with the nitrogen atom, form a radical of the formula 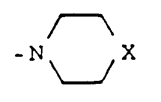 in which
X is O, CH₂ or N-R³,
in which
R³ is hydrogen or methyl, or a physiologically tolerated salt.

4. A compound as in one or more of claims 1 to 3 for use as a pharmaceutical.

5. A compound as in one or more of claims 1 to 3 and pyrimidine-4,6-dicarboxylic acid diamide for inhibition of proline hydroxylase and lysine hydroxylase.

6. A compound as in one or more of claims 1 to 3 and pyrimidine-4,6-dicarboxylic acid diamide for use as a fibrosuppressant and immunosuppressant.

7. A pharmaceutical containing a compound of the formula I with tolerated pharmaceutical excipients.

8. The use of a compound of the formula I and pyrimidine-4,6-dicarboxylic acid diamide for preparing a pharmaceutical for influencing the metabolism of collagen and collagen-like substances and the biosynthesis of C1_{q}.

9. The use of a compound of the formula I and pyrimidine-4,6-dicarboxylic acid diamide for preparing a pharmaceutical for the treatment of disturbances in the metabolism of collagen and collagen-like substances or the biosynthesis of C1_{q}.

10. A process for the preparation of pharmaceuticals for influencing the metabolism of collagen and collagen-like substances and the biosynthesis of C1_{q}, which comprises incorporating a compound of the formula I and/or pyrimidine-4,6-dicarboxylic acid diamide into the pharmaceutical.

## Revendications (Revendications pour l'(les) Etat(s) contractant(s) suivant(s): AT, BE, CH, DE, DK, FR, GB, GR, IT, LI, NL, SE)

1. Pyrimidine-4,6-dicarboxamides de formule I 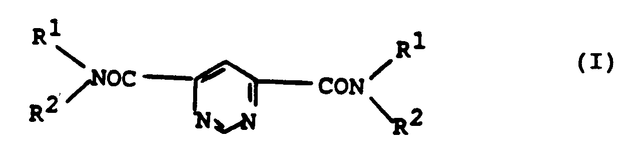 dans laquelle
R¹ représente un radical en C₁-C₁₂, alcényle en C₂-C₁₂ ou alcynyle en C₂-C₁₂, qui sont non substitués ou substitués une fois, ou bien plusieurs fois dans le cas des radicaux alkyle en C₂-C₁₂, alcényle en C₂-C₁₂ et alcynyle en C₂-C₁₂, par
un ou des atomes d'halogène ou groupes hydroxy, cyano, amino, carboxy, alcoxy, alcoxycarbonyle, alkylcarbonyloxy, alkyl- ou dialkylamino, les fragments alkyle comportant de 1 à 4 atomes de carbone, ou par un groupe indolyle ou phényle qui est non substitué ou substitué une, deux ou trois fois par un ou des atomes d'halogène ou groupes nitro, alkyle en C₁-C₄ ou alcoxy en C₁-C₄, dans le cas de substitutions multiples les substituants pouvant également être différents, indépendamment les uns des autres, ou
R¹ représente un radical cycloalkyle en C₅-C₇ saturé, qui est éventuellement soudé à un noyau benzénique, ou
R¹ représente un radical aryle ou hétéroaryle qui est non substitué ou substitué à son tour une, deux ou trois fois par un ou des atomes d'halogène ou groupes nitro, cyano, alkyle en C₁-C₄ ou alcoxy en C₁-C₄, dans le cas de substitutions multiples les substituants pouvant également être différents, indépendamment les uns des autres, ou
R¹, à la condition que R² soit H, représente un groupe amino qui est non substitué ou mono- ou disubstitué par un ou des groupes alkyle en C₁-C₄, phényle ou alkyl(C₁-C₃)-carbonyle, et
R² représente un atome d'hydrogène ou R¹, R² et R¹ étant identiques ou différents,
ou les radicaux R¹ et R² forment ensemble, avec l'atome d'azote, un radical de formule 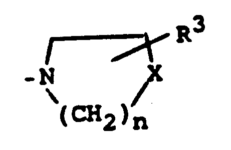 dans laquelle
n va de 1 à 3 et
X représente O, S, CH₂ ou N-R³,
R³ représentant un atome d'hydrogène ou un radical phényle ou alkyle en C₁-C₆, alcényle en C₂-C₆ ou alcynyle en C₂-C₆, ces radicaux phényle, alkyle, alcényle et alcynyle étant non substitués ou substitués une ou plusieurs fois par:
un radical phényle qui, pour sa part, est non substitué ou substitué une ou plusieurs fois par un ou plusieurs substituants choisis parmi les atomes d'halogène et les groupes nitro, cyano, carboxy, hydroxy, méthyle, éthyle, méthoxy, éthoxy et trifluorométhyle, ou
un groupe N(R⁴)₂, R⁴ représentant H ou un groupe alkyle en C₁-C₃, ou
un groupe COOR⁵, R⁵ représentant H ou un groupe alkyle en C₁-C₃, ou
un groupe CON(R⁶)₂ ou CONHR⁶, R⁶ représentant H ou un groupe alkyle en C₁-C₃, ou (R⁶)₂ représentant une chaîne alkylène en C₄-C₆, dans laquelle aucun groupe CH₂ n'est remplacé ou un groupe CH₂ qui n'est pas directement adjacent à l'atome d'azote est remplacé par O, S ou N-R⁴,
ou
R³ représentant un groupe alcoxy(C₁-C₄)-carbonyle ou cycloalkyle en C₃-C₇,
et sels physiologiquement acceptables.

2. Pyrimidine-4,6-dicarboxamides de formule I selon la revendication 1, dans lesquels
R¹ représente un radical alkyle en C₁-C₁₂, qui est non substitué ou substitué une fois, ou bien plusieurs fois dans le cas des radicaux alkyle en C₂-C₁₂, par un ou des groupes phényle, hydroxy, alcoxy, amino, alcoxycarbonyle, alkyl- ou dialkylamino, les fragments alkyle comportant de 1 à 3 atomes de carbone, ou
R¹ représente un radical phényle qui est non substitué ou à son tour substitué une fois par un atome d'halogène ou par un groupe nitro, cyano, méthyle ou méthoxy, ou
R¹, à la condition que R² soit H, représente un radical amino qui est non substitué ou monosubstitué par un groupe alkyle en C₁-C₃, phényle ou alkyl(C₁-C₃)-carbonyle, et
R² représente un atome d'hydrogène, ou dans lesquels les radicaux R¹ et R² forment ensemble, avec l'atome d'azote, un radical de formule 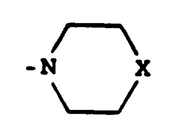 dans laquelle
X représente O, CH₂ ou N-R³,
R³ représentant un atome d'hydrogène ou un groupe alkyle en C₁-C₃,
ainsi que les sels physiologiquement acceptables.

3. Pyrimidine-4,6-dicarboxamides de formule I selon la revendication 1, dans lesquels
R¹ représente un radical alkyle en C₁-C₁₂, qui est non substitué ou substitué une fois-ou bien plusieurs fois dans le cas des radicaux alkyle en C₂-C₁₂, par
un ou des groupes phényle, hydroxy, alcoxy, alcoxycarbonyle ou dialkylamino, les fragments alkyle comportant de 1 à 3 atomes de carbone, ou
R¹ représente le radical phényle, ou
R¹, à la condition que R² soit H, représente un radical amino qui est non substitué ou monosubstitué par le groupe méthylcarbonyle, et
R² représente un atome d'hydrogène,
ou dans lesquels les radicaux R¹ et R² forment ensemble, avec l'atome d'azote, un radical de formule 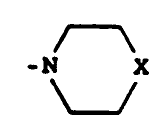 dans laquelle
X représente O, CH₂ ou N-R³,
R³ représentant un atome d'hydrogène ou le groupe méthyle,
et sels physiologiquement acceptables.

4. Procédé pour la préparation des composés de formule I, caractérisé en ce que l'on fait réagir un composé de formule II 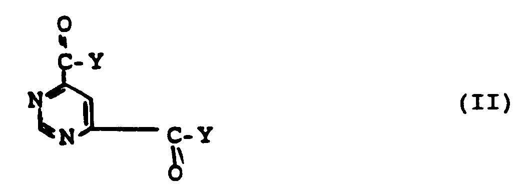 avec un composé de formule III, IV ou V 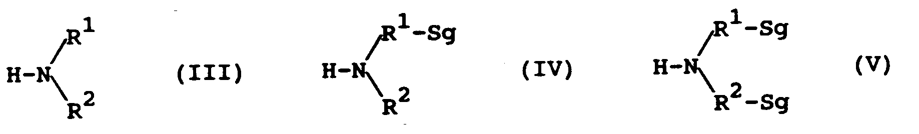 formules dans lesquelles R¹ et R² ont les significations données à propos de la formule I et Y est un atome d'halogène ou un groupe hydroxy ou alcoxy en C₁-C₄, ou forme, conjointement avec le groupe carbonyle, un ester actif ou un anhydride mixte, et Sg est un groupe protecteur,
et on élimine ensuite les groupes protecteurs éventuellement présentsdans le composé de formule I, et on convertit éventuellement les produits de réaction en leurs sels physiologiquement acceptables.

5. Composés selon une ou plusieurs des revendications 1 à 3, pour utilisation en tant que médicaments.

6. Composés selon une ou plusieurs des revendications 1 à 3 et pyrimidine-4,6-dicarboxamide pour l'inhibition de la proline hydroxylase et de la lysine hydroxylase.

7. Composés selon une ou plusieurs des revendications 1 à 3 et pyrimidine-4,6-dicarboxamide, pour utilisation en tant que fibrosuppresseurs et immunosuppresseurs.

8. Médicament contenant un composé de formule I avec des véhicules pharmaceutiquement acceptables.

9. Utilisation de composés de formule I ainsi que du pyrimidine-4,6-dicarboxamide pour la fabrication d'un médicament destiné à avoir un effet sur le métabolisme du collagène et de substances analogues au collagène, ou sur la biosynthèse du C1_{q}.

10. Utilisation de composés de formule I et du pyrimidine-4,6-dicarboxamide pour la fabrication d'un médicament destiné au traitement de troubles du métabolisme du collagène et de substances analogues au collagène, ou de la biosynthèse du C1_{q}.

11. Procédé pour la fabrication de médicaments destinés à agir sur le métabolisme du collagène et de substances analogues au collagène, ou sur la biosynthèse du C1_{q}, caractérisé en ce que l'on incorpore dans le médicament un composé de formule I et/ou du pyrimidine-4,6-dicarboxamide.

## Revendications (Revendications pour l'(les) Etat(s) contractant(s) suivant(s): ES)

1. Procédé pour la préparation de composés de formule I 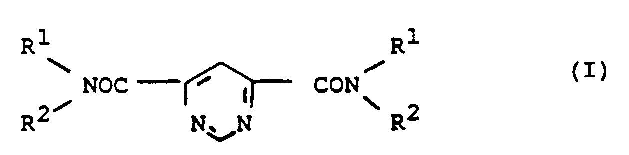 dans laquelle
R¹ représente un radical en C₁-C₁₂, alcényle en C₂-C₁₂ ou alcynyle en C₂-C₁₂, qui sont non substitués ou substitués une fois, ou bien plusieurs fois dans le cas des radicaux alkyle en C₂-C₁₂, alcényle en C₂-C₁₂ et alcynyle en C₂-C₁₂, par
un ou des atomes d'halogène ou groupes hydroxy, cyano, amino, carboxy, alcoxy, alcoxycarbonyle, alkylcarbonyloxy, alkyl- ou dialkylamino, les fragments alkyle comportant de 1 à 4 atomes de carbone, ou par un groupe indolyle ou phényle qui est non substitué ou substitué une, deux ou trois fois par un ou des atomes d'halogène ou groupes nitro, alkyle en C₁-C₄ ou alcoxy en C₁-C₄, dans le cas de substitutions multiples les substituants pouvant également être différents, indépendamment les uns des autres, ou
R¹ représente un radical cycloalkyle en C₅-C₇ saturé, qui est éventuellement soudé à un noyau benzénique, ou
R¹ représente un radical aryle ou hétéroaryle qui est non substitué ou substitué à son tour une, deux ou trois fois par un ou des atomes d'halogène ou groupes nitro, cyano, alkyle en C₁-C₄ ou alcoxy en C₁-C₄, dans le cas de substitutions multiples les substituants pouvant également être différents, indépendamment les uns des autres, ou
R¹, à la condition que R² soit H, représente un groupe amino qui est non substitué ou mono- ou disubstitué par un ou des groupes alkyle en C₁-C₄, phényle ou alkyl(C₁-C₃)-carbonyle, et
R² représente un atome d'hydrogène ou R¹, R² et R¹ étant identiques ou différents,
ou les radicaux R¹ et R² forment ensemble, avec l'atome d'azote, un radical de formule 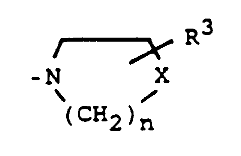 dans laquelle
n va de 1 à 3 et
X représente O, S, CH₂ ou N-R³,
R³ représentant un atome d'hydrogène ou un radical phényle ou alkyle en C₁-C₆, alcényle en C₂-C₆ ou alcynyle en C₂-C₆, ces radicaux phényle, alkyle, alcényle et alcynyle étant non substitués ou substitués une ou plusieurs fois par:
un radical phényle qui, pour sa part, est non substitué ou substitué une ou plusieurs fois par un ou plusieurs substituants choisis parmi les atomes d'halogène et les groupes nitro, cyano, carboxy, hydroxy, méthyle, éthyle, méthoxy, éthoxy et trifluorométhyle, ou
un groupe N(R⁴)₂, R⁴ représentant H ou un groupe alkyle en C₁-C₃, ou
un groupe COOR⁵, R⁵ représentant H ou un groupe alkyle en C₁-C₃, ou
un groupe CON(R⁶)₂ ou CONHR⁶, R⁶ représentant H ou un groupe alkyle en C₁-C₃, ou (R⁶)₂ représentant une chaîne alkylène en C₄-C₆, dans laquelle aucun groupe CH₂ n'est remplacé ou un groupe CH₂ qui n'est pas directement adjacent à l'atome d'azote est remplacé par O, S ou N-R⁴,
ou
R³ représentant un groupe alcoxy(C₁-C₄)-carbonyle ou cycloalkyle en C₃-C₇,
et des sels physiologiquement acceptables, caractérisé en ce que l'on fait réagir un composé de formule II 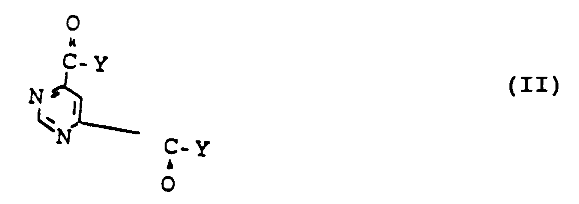 avec un composé de formule III, IV ou V 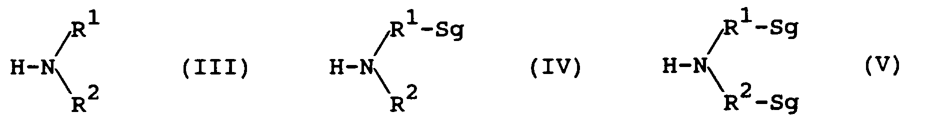 formules dans lesquelles R¹ et R² ont les significations données à propos de la formule I et Y est un atome d'halogène ou un groupe hydroxy ou alcoxy en C₁-C₄, ou forme, conjointement avec le groupe carbonyle, un ester actif ou un anhydride mixte, et Sg est un groupe protecteur, et on élimine ensuite les groupes protecteurs éventuellement présents dans le composé de formule I, et on convertit éventuellement les produits de réaction en leurs sels physiologiquement acceptables.

2. Procédé selon la revendication 1, dans lequel on prépare des composés de formule I dans lesquels
R¹ représente un radical alkyle en C₁-C₁₂, qui est non substitué ou substitué une fois, ou bien plusieurs fois dans le cas des radicaux alkyle en C₂-C₁₂, par
un ou des groupes phényle, hydroxy, alcoxy, amino, alcoxycarbonyle, alkyl- ou dialkylamino, les fragments alkyle comportant de 1 à 3 atomes de carbone, ou
R¹ représente un radical phényle qui est non substitué ou à son tour substitué une fois par un atome d'halogène ou par un groupe nitro, cyano, méthyle ou méthoxy, ou
R¹, à la condition que R² soit H, représente un radical amino qui est non substitué ou monosubstitué par un groupe alkyle en C₁-C₃, phényle ou alkyl(C₁-C₃)-carbonyle, et
R² représente un atome d'hydrogène, ou dans lesquels les radicaux R¹ et R² forment ensemble, avec l'atome d'azote, un radical de formule 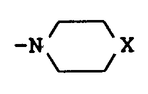 dans laquelle
X représente O, CH₂ ou N-R³,
R³ représentant un atome d'hydrogène ou un groupe alkyle en C₁-C₃
et les sels physiologiquement acceptables.

3. Procédé selon la revendication 1, dans lequel on prépare des composés de formule I dans lesquels
R¹ représente un radical alkyle en C₁-C₁₂, qui est non substitué ou substitué une fois, ou bien plusieurs fois dans le cas des radicaux alkyle en C₂-C₁₂, par un ou des groupes phényle, hydroxy, alcoxy, alcoxycarbonyle ou dialkylamino, les fragments alkyle comportant de 1 à 3 atomes de carbone, ou
R¹ représente un radical phényle, ou
R¹, à la condition que R² soit H, représente un radical amino qui est non substitué ou monosubstitué par le groupe méthylcarbonyle, et
R² représente un atome d'hydrogène, dans lesquelles les radicaux R¹ et R² forment ensemble, ou avec l'atome d'azote, un radical de formule 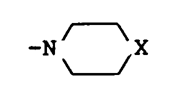 dans laquelle
X représente O, CH₂ ou N-R³,
R³ représentant un atome d'hydrogène ou un groupe méthyle,
et les sels physiologiquement acceptables.

4. Composés selon une ou plusieurs des revendications 1 à 3, pour utilisation en tant que médicaments.

5. Composés selon une ou plusieurs des revendications 1 à 3 et pyrimidine-4,6-dicarboxamide pour l'inhibition de la proline hydroxylase et de la lysine hydroxylase.

6. Composés selon une ou plusieurs des revendications 1 à 3 et pyrimidine-4,6-dicarboxamide, pour utilisation en tant que fibrosuppresseurs et immunosuppresseurs.

7. Médicament contenant un composé de formule I avec des véhicules pharmaceutiquement acceptables.

8. Utilisation de composés de formule I ainsi que du pyrimidine-4,6-dicarboxamide pour la fabrication d'un médicament destiné à avoir un effet sur le métabolisme du collagène et de substances analogues au collagène, ou sur la biosynthèse du C1_{q}.

9. Utilisation de composés de formule I et du pyrimidine-4,6-dicarboxamide pour la fabrication d'un médicament destiné au traitement de troubles du métabolisme du collagène et de substances analogues au collagène, ou de la biosynthèse du C1_{q}.

10. Procédé pour la fabrication de médicaments destinés à agir sur le métabolisme du collagène et de substances analogues au collagène, ou sur la biosynthèse du C1_{q}, caractérisé en ce que l'on incorpore dans le médicament un composé de formule I et/ou du pyrimidine-4,6-dicarboxamide.
